# EUROPEAN PATENT APPLICATION

(11) **EP 1 707 599 A1**
(43) Date of publication of application: **04.10.2006**
(21) Application number: 05703785.5
(22) Date of filing: 18.01.2005
(51) Int. Cl.: C09C 1/28, C09C 3/06, C09D 11/00, C09D 5/29, C09D 7/12, C08K 9/04, C08L 101/00

(54) **COLORED BRIGHT PIGMENT**

(30) Priority: 22.01.2004 JP 2004014551; 07.07.2004 JP 2004199968; 29.11.2004 JP 2004343347
(71) Applicant: Nippon Sheet Glass Company, Limited, Tokyo 105-8552 (JP)
(72) Inventor: INO, Juichi, Nippon Sheet Glass Company, Ltd., Tokyo 1058552 (JP); KITAMURA, Takeaki, Nippon Sheet Glass Company, Ltd, Tokyo 1058552 (JP); YANAGASE, Shigeru, Nippon Sheet Glass Company,Ltd, Tokyo 1058552 (JP); KIMURA, Yusuke, Nippon Sheet Glass Company, Ltd., Tokyo 1058552 (JP)
(74) Representative: Teipel, Stephan
(86) International application number: PCT/JP2005/000548
(87) International publication number: WO 2005/071019

(57) **Abstract**

The present invention is to provide a colored bright pigment which is to be incorporated into a coating material, a resin composition, an ink, a cosmetic and the like, and exhibits not only high transparency and brightness but also high chroma and clear color.

The colored bright pigment is **characterized in that** it comprises scaly glass flakes on the surface of which a silica-based coating film containing an organic pigment is coated. It is preferred that a titanium oxide or a metal is coated on the surface of the scaly glass flakes and the silica-based coating film is further coated thereon.

The above colored bright pigment is used after being incorporated into a coating material, a resin composition, an ink, a cosmetic or the like.

## Description

### Technical Field

The present invention relates to a colored bright pigment exhibiting high transparency and brightness. Further, the invention relates to a colored bright pigment having metallic gloss and interference color. Furthermore, the invention is concerned with a coating material, a resin composition, an ink, or a cosmetic, containing the colored bright pigment.

### Background Art

As a bright pigment, there have been previously known scaly aluminum powders, graphite flake particles, scaly glass flakes, silver-coated scaly glass flakes, and mica flake particles coated with a metal oxide such as titanium dioxide or iron oxide.

Since these bright pigments show characteristics in that they reflect the light on the surface and shine brilliantly, they can be used for coating materials, inks, resin compositions, cosmetics, or the like. When the bright pigment is applied to a coating material, an ink or a resin composition, an unique, diversified appearance with excellent decorative properties is given on the surface of the coated products, the printed products or the molded products, respectively, due to a combination with the tone of their foundation.

Furthermore, as a bright pigment with metallic gloss, there have also been known an aluminum powder, a product obtained by pulverizing a foiled resin coated with a metal, and a mica powder coated with a metal. Since these bright pigments have metallic gloss, strong brightness was given by them, resulting in the excellent appearance design.

Therefore, the bright pigments have a wide usage in coating materials used for coating automobiles, motorcycles, OA (office automation) machines, cellular phones, domestic electric appliances; inks used for various printing matters or writing materials; and cosmetics.

Particularly, scaly base materials coated with titanium dioxide or iron oxide exhibit diverse interference colors, depending on the thickness of the coating layer (see, for example, JP 10-101957-A). However, it was difficult to produce a clear color tone by color development of such interference different from color development occurred in the light absorption of organic pigments.

Accordingly, JP 7-133211-A discloses a colored cosmetic pigment which is obtained by coating a metal oxide gel containing a pigment on the surface of an inorganic base material having a smooth surface. Also, JP 2001-152049-A discloses a colored pigment coated with a metal oxide gel using an inorganic pigment.

On the other hand, although a powder coated with a metal powder or with a metal can exhibit high brightness, its design was poor due to the achromatic color. Accordingly, in order to obtain a chromatic color, there has been employed a method wherein a colored pigment was mixed with a powder which was coated with a metal powder or a metal. However, there was a problem that it was difficult to obtain a clear tone, since the color of a powder coated with an achromatic metal powder or an achromatic metal was emphasized.

Accordingly, JP 6-92546-B discloses a method for coloring which comprises the adhesion of a pigment to an individual aluminum flake by the resin coating method.

### Disclosure of the Invention

### Problems to be Solved by the Invention

Since the technology disclosed in JP 7-133211-A mentioned above relates to a cosmetic pigment, the inorganic material practically used therein can be a mica. However, the outward appearance color of the mica is almost white in many cases, and thus a clear color tone could not be obtained even after the mica was coated with a metal oxide gel containing a pigment.

Furthermore, in the technology disclosed in JP 7-133211-A, it was difficult to obtain a sufficient adhesion amount of pigments, resulting in failure to obtain a clear color tone. In addition, even if it was possible to increase an adhesion amount of the pigment, there was a problem that the pigment was liable to be separated and the steps were complicated and troublesome.

Accordingly, it is an object of the present invention to provide a bright pigment exhibiting high transparency and brightness as well as high chroma and clear color. Another object of the present invention is to provide a coating material, a resin composition, an ink, and a cosmetic, with use of this bright pigment.

### Means for Solving the Problems

As a result of various studies by the inventors of the present invention, they have found that a clear color exhibiting high transparency and brightness, and high chroma was obtained by coating the surface of a base material of scaly glass flakes having an optional coating layer on their surface, with a silica-based coating film containing a pigment. The inventors of the present invention have further studied, and completed the present invention.

That is, the present invention relates to:
(1) a colored bright pigment comprising a base material of scaly glass flakes on the surface of which a coating layer is optionally formed, wherein the surface of said base material is coated with a silica-based coating film containing a pigment,
(2) the colored bright pigment according to the above (1), wherein the pigment is an inorganic pigment or an organic pigment,
(3) the colored bright pigment according to the above

(1) or (2), wherein the pigment has an average particle size of 10 nm to 1 µm,
(4) the colored bright pigment according to any one of the above (1) to (3), wherein the base material is scaly glass flakes on the surface of which a coating film layer is formed,
(5) the colored bright pigment according to the above (4), wherein the coating film layer is comprised of at least one metal selected from the group consisting of gold, silver, platinum, palladium, titanium, cobalt and nickel and/or an alloy thereof,
(6) the colored bright pigment according to the above (4), wherein the coating film layer is comprised of at least one metal oxide selected from the group consisting of titanium oxide, iron oxide and zirconium oxide,
(7) the colored bright pigment according to any one of the above (1) to (6), wherein the silica-based coating film is a matrix comprising substantially only silica,
(8) a coating material comprising the colored bright pigment according to any one of the above (1) to (7),
(9) a resin composition comprising the colored bright pigment according to any one of the above (1) to (7),
(10) an ink comprising the colored bright pigment according to any one of the above (1) to (7), and
(11) a cosmetic comprising the colored bright pigment according to any one of the above (1) to (7).

### Effect of the Invention

The colored bright pigment of the present invention is coated with a silica-based coating film comprising scaly glass flakes containing a pigment. Unlike a mica, since the scaly glass flakes are transparent and have a smooth surface, a pigment comprised of this base material exhibits high transparency and brightness. In addition, since the color development of the pigment of the present invention is not fundamentally based on interference color, but based on the pigment, a clear color with high chroma can be obtained in the present invention.

Thus, deep tone which has previously been unknown can be realized by applying these colored bright pigments to coating materials or resin compositions. For example, a face constituting a curved surface is frequently observed from a small angle to the face in many cases. Even in such a case, a clear color tone is not spoiled in coating materials or resin compositions to which the colored bright pigment of the present invention is applied, and thus the inventive colored bright pigment is preferred.

Furthermore, when using scaly glass flakes which are coated with a metal oxide (especially titanium oxide) as a base material, there can be obtained a bright pigment wherein the color of the pigment (especially organic pigments) is different from that of the scaly glass flakes exhibiting interference color. In this case, the so-called flip-flop that changes a color tone can be achieved when viewed from different angles, resulting in making it possible to provide products with various color tones.

### Brief Explanation of the Drawing

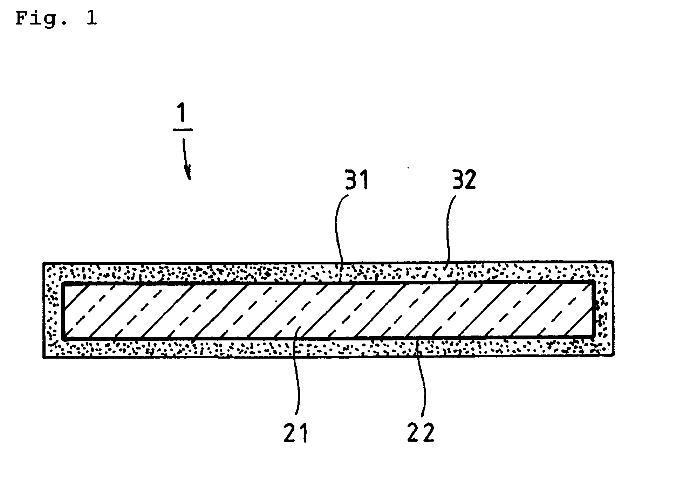

Figure 1 is a schematic cross-section showing an example of the colored bright pigment of the present invention.

### Explanation of the Symbols

| | |
|---|---|
| 1: | Colored bright pigment |
| 21: | Scaly glass flakes |
| 22: | Coating layer |
| 31: | Silica-based coating film |
| 32: | Pigment |

### Best mode for carrying out the invention

The colored bright pigment of the present invention is characterized in that a base material of scaly glass flakes having optionally a coating layer on the surface is coated with a silica-base coating film containing a pigment.

### (Base Material)

As the base material for the present invention, it is preferable to use scaly glass flakes with highly smooth surface and high transparency. A coating film layer may be formed on the surface of these scaly glass flakes. Since a metallic gloss is given when the scaly glass flakes are coated with a metal, it is preferred to use such metal-coated scaly glass flakes. Example of the metal is at least one metal selected from the group consisting of gold, silver, platinum, palladium, titanium, cobalt and nickel and/or an alloy thereof. Furthermore, since interference color is exhibited when scaly glass flakes are coated with a metal oxide (especially titanium oxide), such coated scaly glass flakes are preferably used. The metal oxide can be preferably at least one metal oxide selected from the group consisting of titanium oxide, iron oxide and zirconium oxide, and among them, titanium oxide is more preferable.

The scaly glass flakes used in the present invention may be produced by the "blow" method. The "blow" method comprises melting a raw cullet; taking the melted glass continuously out from circular slits at the time when a gas such as air or the like is blown from the blow nozzles which are installed on the inner side of the circular slits; forming a balloon while swelling the melted glass, followed by drawing; and crushing the thinned glass, thereby to give scaly glass flakes.

The surface of the obtained scaly glass flakes retains fire-polished surface with smoothness as is at the time of melting molding. The said scaly glass flakes can reflect the light well because of its smooth surface. It is preferable to incorporate these scaly glass flakes into a coating material or a resin composition, because a highly brightness can be obtained. As scaly glass flakes like these, for example, MICROGLAS (registered trade mark) R-GLASFLAKE (registered trade mark) R-series (RCF-160, REF-160, RCF-015, REF-015) are commercially available from Nippon Sheet Glass Co., Ltd.

There is no particular limitation on the method for coating scaly glass flakes with a metal, and any one of the known methods can be used.

As the scaly glass flakes coated with a metal and exhibiting brightness, METASHINE (registered trade mark) RPS series such as MC5480PS, MC5230PS, MC5150PS, MC5090PS, MC5030PS, MC2080PS, ME2040PS, ME2025PS, MC5480NS, MC5230NS, MC5150NS, MC5090NS, MC5030NS, MC5480NB, MC5230NB, MC5150NB, MC5090NB, MC5030NB, MC1040NB, and MC1020NB are commercially available from Nippon Sheet Glass Co., Ltd.

There is no particular limitation on the method for coating scaly glass flakes with a metal oxide, and the known methods such as spattering method, sol-gel method, CVD method and LPD method can be used. In the case where a particle base material like scaly glass flakes is coated with a metal oxide, it is preferred to use the LPD method where the metal oxide is precipitated on the surface from the metal salt. Also, in order to exhibit interference color, a metal oxide having a high diffraction efficiency is preferable. For example, in the case of titanium oxide, a precipitation method utilizing neutralization reaction is most suitable, such as the method wherein the rutile form of titanium dioxide can be directly precipitated on the surface of the scaly glass flakes. More particularly, as disclosed in JP 2001-31421-A by the inventors of the present invention, it is a method which comprises precipitating the titanium dioxide in rutile crystal form from a titanium-containing solution at 55°C to 85°C within a pH range of not more than 1.3.

As the scaly glass flakes which are coated with a metal oxide and exhibit interference color, for example, METASHINE (registered trade mark) RRC series such as MC5090RS, MC5090RY, MC5090RR, MC5090RB, MC5090RG, MC1080RS, MC1080RY, MC1080RR, MC1080RB, MC1080RG, MC1040RS, MC1040RY, MC1040RR, MC1040RB, MC1040RG, MC1020RS, MC1020RY, MC1020RR, MC1020RB, MC1020RG, MC5090FG, MC5090FR, MC5090FB, and MC5090FC are commercially available from Nippon Sheet Glass Co., Ltd.

There is no particular limitation on the average particle size and the average thickness of the scaly glass flakes on the surface of which a coating layer is optionally formed, though they differ depending on the application purpose. Generally, the average particle size is preferably 1 to 500 µm, and the average thickness is preferably 0.1 to 10 µm. In the case where the particle size is too large, the scaly glass flakes are broken at the time when the bright pigment is incorporated into a coating material or a resin composition. Particularly, in the case of the scaly glass flakes which are coated with titanium oxide, alkali components contained therein become diffused because of the exposure of the cross-section.

On the other hand, when the particle size is too small, the plane of the bright pigment in the coating material or resin composition is oriented in a random direction to weaken the reflected light emitted by individual particles, resulting in loss of brightness.

When the bright pigment is used for inks, its particle size is preferably small, and the average particle size of the scaly glass flakes on the surface of which a coating layer may be formed is preferably 1 to 40 µm and the average thickness is preferably 0.5 to 3 µm.

In order to impart or improve chemicals-resistance, especially alkali-resistance of the scaly glass flakes used in the present invention wherein a coating layer may be formed on its surface, the most upper surface may be coated with zirconium oxide, aluminum oxide, zinc oxide or the like. The coating with zirconium oxide is performed by the known method such as liquid phase deposition.

### (Pigments)

The pigment used in the present invention may be any organic pigments or inorganic pigments. Although there is no particular limitation on the size of the pigments used, the average particle size is preferably 10 nm to 1 µm. In the case of the average particle size of less than 10 nm, the durability of the pigment becomes extremely worsened. On the other hand, when the average particle size exceeds 1 µm, the hiding power by the pigment becomes larger, resulting in loss of brightness. Therefore, the tone is weakened, and if the coating film is thin, the surface smoothness of the coating film is lost.

The content of the pigment is preferably not more than 30% by mass, more preferably less than 10% by mass, most preferably 0.1 to 3.0% by mass, relative to the mass of the base material (scaly glass flakes optionally having a coating layer).

There is no particular limitation on the kind of inorganic pigments, but, for example, the following pigments are preferably used. The name of the pigments is expressed according to Color Index Generic Name (hereinafter abbreviated as C.I.GN.).

That is, such pigments include red iron oxide (C.I.GN. Pigment Red 101), iron black (C.I.GN. Pigment Black 11), titanium yellow (C.I.GN. Pigment Yellow 157, Yellow 53, Brown 24), zinc-iron brown (C.I.GN. Pigment Yellow 119, Brown 33), titanium cobalt green (C.I.GN. Pigment Green 50), cobalt green(C.I.GN. Pigment Green 26), cobalt blue (C.I.GN. Pigment Blue 28), copper-iron black (C.I.GN. Pigment Black 26), zinc sulfide (C.I.GN. Pigment White 7), barium sulfate (C.I.GN. Pigment White 21), ultramarine blue (C.I.GN. Pigment Blue 29), calcium carbonate (C.I.GN. Pigment White 18), cobalt violet (C.I.GN. Pigment Violet 14), yellow iron oxide (C.I.GN. Pigment Yellow 42, Yellow 43), carbon black (C.I.GN. Pigment Black 7), ferrocyan (C.I.GN. Pigment Blue 27), and the like.

Although there is no particular limitation on the kind of organic pigments, there are exemplified phthalocyanin pigments, insoluble azo pigments, azo lake pigments, anthraquinone pigments, quinacridone pigments, dioxazine pigments, diketopyrrolopyrrole pigments, anthrapyridine pigments, anthanthrone pigments, indanthrone pigments, flavanthrone pigments, perinone pigments, perilene pigments, thioindigo pigments, or the like.

Among the C.I.GN, in the case where weather-resistance is required in the application to automobiles, it is preferable to use the organic pigments such as Pigment Green 36, Pigment Red 179, Pigment Blue 15, and the like.

As the organic pigments used for cosmetics, there are firstly exemplified red pigments including FD & C red No.2, FD & C Red No.3, FD & C Red No.102, FD & C Red No.104, FD & C Red No.105, FD & C Red No.106, FD & C Red No.201, FD & C Red No.202, FD & C Red No.203, FD & C Red No.204, FD & C Red No.205, FD & C Red No.206, FD & C Red No.207, FD & C Red No.208, FD & C Red No.213, FD & C Red No.214, FD & C Red No.215, FD & C Red No.218, FD & C Red No.219, FD & C Red No.220, FD & C Red No.221, FD & C Red No.223, FD & C Red No.225, FD & C Red No.226, FD & C Red No.227, FD & C Red No.228, FD & C Red No.230(1), FD & C Red No.230(2), FD & C Red No.231, FD & C Red No.232, FD & C Red No.405, and the like.

Also, as the yellow pigments, there are exemplified FD & C Yellow No.4, FD & C Yellow No.5, FD & C Yellow No.201, FD & C Yellow No.202-1, FD & C Yellow No.202-2, FD & C Yellow No.203, FD & C Yellow No. 204, FD & C Yellow No.205, FD & C Yellow No.401, FD & C Yellow No.402, FD & C Yellow No.403, FD & C Yellow No. 404 , FD & C Yellow No.405, FD & C Yellow No.406, FD & C Yellow No.407.

As the green pigments, there are exemplified FD & C Green No.3, FD & C Green No.201, FD & C Green No.202, FD & C Green No.204, FD & C Green No.205, FD & C Green No.401, FD & C Green No.402, and the like.

Furthermore, as the blue pigments, there are exemplified FD & C Blue No.1, FD & C Blue No.2, FD & C Blue No.201, FD & C Blue No.202, FD & C Blue No.203, FD & C Blue No.204, FD & C Blue No.205, FD & C Blue No. 403, FD & C Blue No. 404, and as the orange pigments, there are exemplified FD & C Orange No.201, FD & C Orange No.203, FD & C Orange No.204, FD & C Orange No.205, FD & C Orange No.206, FD & C Orange No.207, FD & C Orange No.401, FD & C Orange No.402, FD & C Orange No.403. In addition to these pigments, there are further exemplified FD & C Brown No.201, FD & C Purple No.201, FD & C Purple No.401, and FD & C Black No.401.

When the scaly glass flakes coated with titanium oxide exhibit interference color, there is no particular limitation on the combination of the interference color of the scaly glass flakes with the color of the pigments. If the interference color is made closer to the color of the pigment (especially organic pigments), it is possible to increase the depth of the color, leading to the formation of preferable color.

When the scaly glass flakes exhibiting interference color is applied as a coating film without being coated with a silica-based coating film containing a pigment, the interference color looks somewhat different and also looks opaque white due to light scattering, when viewed, in particular, at a low angle to the coated surface.

However, when the scaly glass flakes exhibiting interference color is coated with a silica-based coating film containing a pigment and then used as a coating film, not only different interference color but also white opaqueness was not observed even when viewed from a small angle to the coated surface. The color tone of this coating is stable when it is viewed even from any angle, and thus deep tone of the color can be realized.

By using the interference color which is different from the color of the pigment (especially organic pigments), the so-called flip-flop color, i.e., the color looking different depending on the viewing angle, can be achieved. In the flip-flop of the bright pigment utilizing only the interference color, the color tone varies on the order of the wave length of light, while free color change can be realized in the flip-flop wherein the interference color and the pigment color are different from each other, for example, blue and yellow, or red and green.

### (Silica-based coating film)

The pigment is contained in a silica-based coating film and coated on the surface of a base material (that is, scaly glass flakes wherein a coating layer may be formed on its surface). The silica-based coating film can be easily applied on the surface of granular scaly glass flakes by, for example, the sol-gel method. Also, the silica-based coating film may be preferably used as a matrix wherein a pigment is to be contained in a dispersed state.

The silica-based coating film may comprise silica as a main component, and its matrix may be comprised of substantially only silica. As a component other than silica, titanium or zirconium may be contained therein so as to adjust the refractive index or to impart alkali-resistance.

The silica-based coating film containing a pigment has preferably a thickness of 50 nm to 1 µm. When the thickness is not more than 50 nm, it is not able to contain the pigment in a sufficient amount, and the color tone becomes thinner or the pigment contained therein is apt to be separated.

On the other hand, when the thickness exceeds 1 µm, the color tone becomes thick to lose the brightness, or the pigment-containing layer is apt to be separated. In addition, the production cost becomes more expensive.

### (Production method of silica-based coating film containing pigment)

Production method of the silica-based coating film containing a pigment may be performed by dispersing a base material in a coating solution containing an organometal compound (containing an organosilica compound capable of undergoing hydrolysis/polycondensation) and a pigment, followed by hydrolysis/polycondensation of said organometal compound, so that the surface of the base material is coated with the silica-based coating film. Further, the organometal compound may be comprised of an organosilica compound only. As an organometal compound other than the organosilica compound, it includes, for example, an organotitanium compound, an organozirconium compound, and an organoaluminum compound.

Examples of the organosilica compound contained in the coating solution are tetraalkoxysilanes (e.g. tetramethoxysilane, tetraethoxysilane, tetrapropoxysilane, tetrabutoxysilane, etc.) and methyltrialkoxysilanes (e.g. methyltrimethoxysilane, methyltriethoxysilane, methyltripropoxysilane, methyltributoxysilane, etc.).

Among these organosilica compounds, it is preferable to use a relatively low molecular weight compound, for example, a tetraalkoxysilane having an alkoxy group of 3 or less carbon atom(s), because such tetraalkoxysilane may form a fine coating. Further, among these polymers of the tetraalkoxysilane, it is also preferred to use those having the average polymerization degree of not more than 5.

The coating solution comprises 0.5 to 20 mass% of an organosilica compound and 0.1 to 5 mass% of a pigment. The coating solution further comprises water for the hydrolysis reaction. For example, the mixing ratio (molar ratio) of silicon alkoxide being an organosilica compound and water is preferably 1 : 25 to 100. By changing the content ratio of the organosilica compound and the pigment, the ratio of silica and pigment in the final coating may be adjusted.

The coating solution may further comprise a solvent. Examples of the solvent include organic solvents such as hydrocarbons (e.g. hexane, toluene, cyclohexane, etc.); halogenated hydrocarbons (e.g. methylene chloride, carbon tetrachloride, trichloroethylene, etc.); ketones (e.g. acetone, methyl ethyl ketone, etc.); nitrogen-containing compounds (e.g. diethylamine, etc.); alcohols; and esters (e.g. ethyl acetate, etc.). Among these solvents, an alcohol solvent including, for example, methyl alcohol, ethyl alcohol, 1-propyl alcohol, 2-propyl alcohol, butyl alcohol, and amyl alcohol is preferably used. Among these alcohols, examples of the alcohols preferably used are a linear saturated monovalent alcohol having 3 or less carbon atom(s), such as methyl alcohol, ethyl alcohol, 1-propyl alcohol, and 2-propyl alcohol, because their evaporation velocity at normal temperature is large.

The coating solution further comprises a catalyst. The type of the catalyst is preferably an alkaline catalyst such as ammonium hydroxide, ammonia, sodium hydroxide, and the like. The pH of the coating solution is usually 10 to 14.

As the catalyst used in the sol-gel method, an acid catalyst such as hydrochloric acid, hydrofluoric acid, nitric acid, acetic acid and the like is frequently used. However, like the scaly glass flakes used in the present invention, when a silica-based coating film is applied to a granular base material, the particles agglomerate each other and thus it is preferred to use an alkaline catalyst in the present invention.

A base material (i.e. scaly glass flakes wherein a coating film may be formed on the surface) in 1 to 60% by mass is dispersed in the coating solution, and the dispersion is allowed to stand for 1 to 15 hours so that the organosilicon compound is hydrolyzed and polycondensed. As a result, there is produced a silica-based coating film containing a dispersed pigment wherein a coating of 50 nm to 1 µm thickness is formed on the whole surface of the base material. Then, this product is washed, filtered and dried. The drying is preferably performed at room temperature to 200°C for 3 to 5 hours.

### (Use)

Since the colored bright pigment of the present invention is highly safe and stable, and exhibits pearl gloss and interference rainbow color, it can be applied to various uses such as resin compositions, inks, coating materials, cosmetics and the like.

For example, the resin composition is applied to cosmetic containers, food containers, wall coverings, floor coverings, home appliances, accessories, stationeries, toys, bathtubs, bath goods, footwears, sports goods, toilet articles, or the like.

The application to inks includes wrapping packages, packaging papers, wall papers, clothes, decorative sheets, textiles, various films, labels, and the like.

The coating materials can be applied to outer coating for automobiles, motorcycles or bicycles; building materials; tiles; furnitures; household goods; containers; office supplies; sports goods; or the like.

The cosmetic application includes facial cosmetics, make-up cosmetics, hair cosmetics, and the like. Among these cosmetics, the colored bright pigment is preferably used especially in foundations such as cosmetic foundation and face powder, or in make-up cosmetics such as eye shadow, brusher, nail enamel, eyeliner, mascara, rouge, fancy powder, and the like. There is no particular limitation on the form of cosmetics, and example of such form includes powder, cake, pencil, stick, ointment, liquid, emulsion, cream, and the like.

As other applications, the colored bright pigment is applied to water color, oil color, real leather, synthetic leather, textile printing, button, lacquer, ceramic wear, or the like.

Hereinafter, the present invention is specifically explained by way of Examples wherein Examples 1-1 to 1-3 are those using an inorganic pigment and Examples 2-1 to 2-4 are those using an organic pigment.

### Example 1-1

Tetraethoxysilane (15 ml), ethyl alcohol (300 ml) and purified water (60 ml) were mixed. To the mixture was added a solution (0.8 g) containing fine particles (average particle size of about 70 nm) of an inorganic pigment, Cobalt Blue (C.I.GN.Pigment Blue 28) in 40% by mass, and the mixture was mixed to make a coating solution.

Then, silver-coated glass flakes (30 g)(MEG020PS, manufactured by Nippon Sheet Glass Co., Ltd.) were added to the above coating solution, and the mixture was stirred and mixed in an agitator. The silver-coated glass flakes have 20 µm in average particle size and 0.7 µm in average thickness, and exhibit metallic gloss. Thereafter, an aqueous ammonium hydroxide solution (14 ml) of 25% concentration was added to the coating solution, and the resulting mixture was stirred and mixed for 2 to 3 hours so that dehydrative polycondensation reaction takes place. By this reaction, a silica coating film containing an inorganic pigment is uniformly precipitated on the surface of the glass flakes in the coating solution. Subsequently, the reaction solution was filtered and washed, and this procedure was repeated several times, dried, and finally heated at 180°C for 2 hours, thereby to obtain a bright pigment coated with a silica coating film (thickness:100 nm) containing an inorganic pigment. The bright pigment exhibited a blue color with metallic gloss.

Fig. 1 shows a schematic cross-section of the bright pigment thus obtained. The colored bright pigment 1 of the present invention is characterized in that coating layer 22 is formed on scaly glass flakes 21 of a base material, the coating layer in this Example is comprised of a metal, i.e., a silver-coated layer, and further 32 is a pigment, that is, an inorganic pigment in this Example, i.e. cobalt blue. The base material is coated with a silica-based coating film 31 containing this pigment in the dispersed state.

### Example 1-2

In a similar manner to Example 1-1 except that fine particles of an inorganic pigment (carbon black : C.I.GN. Pigment Black 7) were used in place of fine particles of an inorganic pigment (cobalt blue :C.I.GN. Pigment Blue 28) in Example 1-1, a bright pigment was prepared. That is, by this procedure, a bright pigment coated with a silica coating film (100 nm in thickness) containing an inorganic pigment was obtained. This bright pigment exhibited a black color with metallic gloss.

### Example 1-3

Tetraethoxysilane (4 ml), ethyl alcohol (75 ml) and purified water (15 ml) were mixed. To the mixture was added a solution (0.75 g) containing fine particles (average particle size: about 100 nm) of an inorganic pigment, Cobalt Blue (C.I.GN.Pigment Blue 28) in 10% by mass, and the mixture was mixed to make a coating solution.

Then, nickel-coated scaly glass flakes (30 g) (MC5090NS, manufactured by Nippon Sheet Glass Co., Ltd.) were added to the above coating solution, and the mixture was stirred and mixed in an agitator. The nickel-coated scaly glass flakes have 90 µm in average particle size and 5.0 µm in average thickness, and exhibit metallic gloss. Thereafter, an aqueous ammonium hydroxide solution (0.9 ml) of 25% concentration was added to the coating solution, and the resulting mixture was stirred and mixed for 2 to 3 hours so that dehydrative polycondensation reaction takes place. By this reaction, a silica coating film containing an inorganic pigment is uniformly precipitated on the surface of the scaly glass flakes in the coating solution. Subsequently, the reaction solution was filtered and washed, and this procedure was repeated several times, dried, and finally heated at 180°C for 2 hours, thereby to obtain a bright pigment coated with a silica coating film(thickness:100 nm) containing an inorganic pigment. The bright pigment exhibited a black color with metallic gloss.

### Comparative Example 1-1

The silver-coated scaly glass flakes in Example 1-1 were used as a bright pigment without being coated with a silica-based coating containing a pigment.

### Comparative Example 1-2

In a similar manner to Example 1-1, a bright pigment except that a commercially available aluminum powder was used in place of the silver-coated scaly glass flakes in Example 1-1.

### (Evaluation of bright pigment)

The bright pigments obtained in Example 1-1 to 1-3 and Comparative Examples 1-1 to 1-2 were weighed in each 1 g, and mixed with an acryl resin (49 g as a solid amount)(Acryl Autoclear Super; manufactured by Nippon Paint Co., Ltd.). The mixture was stirred well with a paint shaker to obtain a coating solution. The mixed solution was applied on charts for identifying hiding-power of paint with an applicator, and the charts were allowed to stand at normal temperature for sufficient drying to make a coating film.

The design of the coating film was evaluated by 5 panelists for sensory test. The sensory test was performed in the daytime sunlight, and brightness and clearness of the color were evaluated. The comprehensive evaluation results by the five panelists were summarized below.

### With respect to Example 1-1

The coating film showed strong brightness due to metallic gloss, and a clear and deep blue color was observed in the film. The design was not lost because of no occurrence even when viewed from a small angle to the coated surface.

### With respect to Example 1-2

The coating film showed strong brightness due to metallic gloss, and a clear and deep black color was observed in the film. The design was not lost because of no occurrence of white opaqueness even when viewed from a small angle to the coated surface.

### With respect to Example 1-3

The coating film showed strong brightness due to metallic gloss, and a clear and deep black color was observed in the film. The design was not lost because of no occurrence of white opaqueness even when viewed from a small angle to the coated surface.

### With respect to Comparative Example 1-1

A bright color with metallic gloss was observed. When viewed from a small angle, a clear color was observed without the occurrence of white opaqueness.

### With respect to Comparative Example 1-2

Low bright and scattering in white was observed compared to Example 1-1 or Example 1-3, though the bright color with metallic gloss was observed. The degree of white opaqueness was strong when viewed from a small angle to the coated surface.

By investigation on the results of the above Examples and Comparative Examples, the following was revealed.

Comparison between Example 1-1 and Comparative Example 1-1 revealed that the original brightness due to the metallic gloss was not lost by applying a silica coating film containing an inorganic pigment, and a deep color tone could be retained.

In addition, comparison between Example 1-1 and Comparative Example 1-1 revealed that low bright and scattering in white was observed when using a silver-coated aluminum as a base material for bright pigments. Especially, when viewed from a small angle to the coated surface, the degree of white opaqueness becomes stronger, resulting in very poor color tone.

### Example 2-1

Tetraethoxysilane (15 ml), ethyl alcohol (300 ml) and purified water (60 ml) were mixed. To the mixture was added a solution (3.0 g) containing fine particles (average particle size of about 100 nm) of an organic pigment (C.I.GN.Pigment Blue 15:3) in 10% by mass, and the mixture was mixed to make a coating solution.

Then, titanium dioxide-coated scaly glass flakes (30 g) (MC1020RB, manufactured by Nippon Sheet Glass Co. , Ltd.) were added to the above coating solution, and the mixture was stirred and mixed in an agitator. The scaly glass flakes have 20 µm in average particle size and 1.3 µm in average thickness, and exhibit an interference color with a bright blue color. Thereafter, an aqueous ammonium hydroxide solution (14 ml) of 25% concentration was added to the coating solution, and the resulting mixture was stirred and mixed for 2 to 3 hours so that dehydrative polycondensation reaction takes place. By this reaction, a silica coating film containing an organic pigment is uniformly precipitated on the surface of the scaly glass flakes in the coating solution. Subsequently, the reaction solution was filtered and washed, and this procedure was repeated several times, dried, and finally heated at 180°C for 2 hours, thereby to obtain a bright pigment coated with a silica coating film (thickness: 100 nm) containing an organic pigment. The bright pigment exhibited a blue color.

The schematic cross-section of the obtained bright pigment is the same as Fig. 1 mentioned above, wherein coating layer 22 is titanium dioxide and pigment 32 is an organic pigment.

### Example 2-2

In a similar manner to Example 2-1 except that titanium dioxide-coated scaly glass flakes (MC1020RY: manufactured by Nippon Sheet Glass Co., Ltd.) exhibiting an interference color with yellow brightness was used in place of the titanium dioxide-coated scaly glass flakes in Example 2-1, a bright pigment was produced.

### Example 2-3

In a similar manner to Example 1 except that silver-coated scaly glass flakes (MC2080PS, manufactured by Nippon Sheet Glass) having an average particle size of 80 µm and an average thickness of 1.3 µm and exhibiting metallic gloss was used in place of titanium dioxide-coated scaly glass flakes in Example 2-1, a bright pigment was prepared. Thus, a bright pigment coated with a silica-coating film (100 nm of thickness) containing an organic pigment was obtained. This bright pigment exhibited a blue color with metallic gloss.

### Example 2-4

Tetraethoxysilane (4 ml), ethyl alcohol (75 ml) and pure water (15 ml) were mixed. To the mixture was added a solution (0.75 g) containing fine particles (average particle size: about 100 nm) of an organic pigment (C.I.GN. Pigment Blue 15:3) in 10% by mass, and the mixture was mixed to make a coating solution.

Then, nickel-coated scaly glass flakes (30 g) (MC5090NS, manufactured by Nippon Sheet Glass Co. , Ltd.) were added to the above coating solution, and the mixture was stirred and mixed in an agitator. The scaly glass flakes used have 90 µm in average particle size and 5.0 µm in average thickness, and exhibit a color with metallic gloss. Thereafter, an aqueous ammonium hydroxide solution (0.9 ml) of 25% concentration was added to the coating solution, and the resulting mixture was stirred and mixed for 2 to 3 hours so that dehydrative polycondensation reaction takes place. By this reaction, a silica coating film containing an organic pigment is uniformly precipitated on the surface of the scaly glass flakes in the coating solution. Subsequently, the reaction solution was filtered and washed, and this procedure was repeated several times, dried, and finally heated at 180°C for 2 hours, thereby to obtain a bright pigment coated with a silica coating film (thickness:100 nm) containing an organic pigment. The bright pigment exhibited a blue color with metallic gloss.

### Comparative Example 2-1

The titanium dioxide-coated scaly glass flakes in Example 2-1 was used as a bright pigment without being coated with a silica coating film.

### Comparative Example 2-2

In a similar manner to Example 2-1 except that titanium dioxide-coated mica flakes (Iriodin 221, manufactured by Merck & Co., Inc.: particle size of 5 to 25 µm) exhibiting a blue interference color was used in place of titanium dioxide-coated scaly glass flakes, a bright pigment was prepared.

### Comparative Example 2-3

In a similar manner to Example 2-3 except that a commercially available aluminum powder was used in place of the silver-coated scaly glass flakes in Example 2-3, a bright pigment was prepared.

### (Evaluation of bright pigment)

The bright pigments obtained in Examples 2-1 to 2-4 and Comparative Examples 2-1 to 2-3 were weighed in each 1 g, and mixed with an acryl resin (49 g as a solid weight)(Acryl Autoclear Super; manufactured by Nippon Paint Co. , Ltd.). The mixture was stirred well with a paint shaker to prepare a coating material. The mixed solution was applied on charts for identifying hiding-power of paint with an applicator, and the charts were allowed to stand at normal temperature for sufficient drying to make a coating film.

The design of the coating was evaluated by 5 panelists for sensory test. The sensory test was performed in the daytime sunlight, and evaluation was made on the brightness and clearness of the color. The comprehensive evaluation results by the five panelists were summarized below.

### With respect to Example 2-1

A clear and deep blue color with strong brightness was observed in the coating film. The design was not lost due to the occurrence of white opaqueness and the change of interference color even when viewed from a small angle to the coated surface.

### With respect to Example 2-2

When viewed from right angle to the coated surface, a strongly bright and yellowish interference color was observed. When viewed from a small angle to the coated surface, a clear color was observed without the occurrence of white opaqueness. With respect to Example 2-3

A clear and deep blue color with strong brightness due to metallic gloss was obtained. In addition, even when viewed from a small angle to the coated surface, the design is not lost because of no occurrence of white opaqueness.

### With respect to Example 2-4

A clear and deep blue color with strong brightness due to metallic gloss was obtained. In addition, even when viewed from a small angle to the coated surface, the design is not lost because of no occurrence of white opaqueness.

### With respect to Comparative Example 2-1

When viewed from the right angle to the coated surface, a blue interference color was observed. When viewed from a small angle to the coated surface, the color was changed to a slightly reddish blue color and its clearness was lost due to the occurrence of white opaqueness.

### With respect to Comparative Example 2-2

The change of the color is the same as in Example 2-2. Even when viewed from the right angle to the coated surface, low brightness and scattering in white were observed. Furthermore, when viewed from a small angle to the coated surface, the degree of white opaqueness was strong.

### With respect to Comparative Example 2-3

Although the pigment exhibits brightness due to metallic gloss, its brightness was lower compared to that of Example 2-3 or Example 2-4, and scattering in white was observed. In addition, when viewed from a small angle to the surface to be coated, the degree of cloudiness formation was strong.

By studying the results of the above Examples and Comparative Examples, the following will be elucidated.

It was revealed from the comparison between Example 2-1 and Comparative Example 2-1 that a deep color tone without haze and change in the interference color could be retained by applying a silica coating film containing an organic pigment, even when viewed at a low angle to the coated surface.

From the comparison between Example 2-2 and Comparative Example 2-2, it is found that the brightness is low and scattering in white is observed when using a titanium dioxide-coated mica as a base material for bright pigments. Especially, when viewed from a small angle to the coated surface, the degree of white opaqueness becomes stronger, resulting in very poor color tone.

### Industrial Applicability

The colored pigments of the present invention can be applied in various fields for coating materials, resin compositions, inks, cosmetics, artificial marble products, coated papers, or the like.

## Claims

1. A colored bright pigment comprising a base material of scaly glass flakes on the surface of which a coating layer is optionally formed, wherein the surface of said base material is coated with a silica-based coating film containing a pigment.

2. The colored bright pigment according to claim 1, wherein the pigment is an inorganic pigment or an organic pigment.

3. The colored bright pigment according to claim 1 or claim 2, wherein the pigment has an average particle size of 10 nm to 1 µm.

4. The colored bright pigment according to any one of claims 1 to 3, wherein the base material is scaly glass flakes on the surface of which a coating film layer is formed.

5. The colored bright pigment according to claim 4, wherein the coating film layer is comprised of at least one metal selected from the group consisting of gold, silver, platinum, palladium, titanium, cobalt and nickel, and/or an alloy thereof.

6. The colored bright pigment according to claim 4, wherein the coating film layer is comprised of at least one metal oxide selected from the group consisting of titanium oxide, iron oxide and zirconium oxide.

7. The colored bright pigment according to any one of claims 1 to 6, wherein the silica-based coating film is a matrix comprising substantially only silica.

8. A coating material comprising the colored bright pigment according to any one of claims 1 to 7.

9. A resin composition comprising the colored bright pigment according to any one of claims 1 to 7.

10. An ink comprising the colored bright pigment according to any one of claims 1 to 7.

11. A cosmetic comprising the colored bright pigment according to any one of claims 1 to 7.
